# EUROPEAN PATENT SPECIFICATION

(11) **EP 1 334 108 B1**
(45) Date of publication and mention of the grant of the patent: **15.06.2005**
(21) Application number: 00991478.9
(22) Date of filing: 13.11.2000
(51) Int. Cl.: C07F 9/6512

(54) **ARYL PHOSPHATE DERIVATIVES OF d4T**
ARYLPHOSPHATDERIVATE VON D4T
DERIVES ARYLPHOSPHATE DE D4T

(43) Date of publication of application: 13.08.2003
(73) Proprietor: Parker Hughes Institute, St. Paul, MN 55113 (US)
(72) Inventor: UCKUN, Fatih, M., White Bear Lake, MN 55110 (US)
(74) Representative: Gallagher, Kirk James
(86) International application number: PCT/US2000/042132
(87) International publication number: WO 2002/038576

(56) References cited:
- WO-A-96/29336
- SIDDIQUI, ADAM Q. ET AL: "Design and Synthesis of Lipophilic Phosphoramidate d4T-MP Prodrugs Expressing High Potency Against HIV in Cell Culture: Structural Determinants for in Vitro Activity and QSAR" J. MED. CHEM. (1999), 42(20), 4122-4128 , XP000999173
- MCGUIGAN, CHRISTOPHER ET AL: "Aryl Phosphoramidate Derivatives of d4T Have Improved Anti-HIV Efficacy in Tissue Culture and May Act by an Entirely New Mechanism of Reverse Transcriptase Inhibition" J. MED. CHEM. (1996), 39(8), 1748-53 , XP000578130 cited in the application

## Description

### Field of the Invention

The present invention is directed to aryl phosphate nucleoside derivatives, particularly aryl phosphate derivatives of 2',3'-didehydro-3'-deoxythymidine (hereinafter "d4T"), that exhibit antiviral activity, for example against the human immune deficiency virus (HIV), e.g. as inhibitors of HIV reverse transcriptase.

### Background of the Invention

The spread of AIDS and the ongoing efforts to control the responsible virus are well-documented. One way to control HIV is to inhibit its reverse transcriptase activity (RT). Thus, novel, potent, and selective inhibitors of HIV RT are needed as useful therapeutic agents. Known, potent inhibitors of HIV RT include 5'-triphosphates of 2',3'-dideoxynucleoside ("ddN") analogues. These active RT inhibitors are generated intracellularly by the action of nucleoside kinase and nucleotide kinase. Thus, ddN compounds such as AZT and d4T have been considered to hold much promise in the search for anti-HIV agents.

The rate-limiting step for the conversion of 3'-azido-3'-deoxythymidine (Zidovudine; AZT) to its bioactive metabolite AZT-triphosphate seems to be the conversion of the monophosphate derivative to the diphosphate derivative, whereas the rate-limiting step for the intracellular generation of the bioactive d4T metabolite d4T-triphosphate was reported to be the conversion of the nucleoside to its monophosphate derivative. (Balzarini et al., 1989, *J.Biol. Chem*. 264:6127; McGuigan et al., 1996, *J. Med. Chem*. 39:1748). The following mechanism has been proposed:

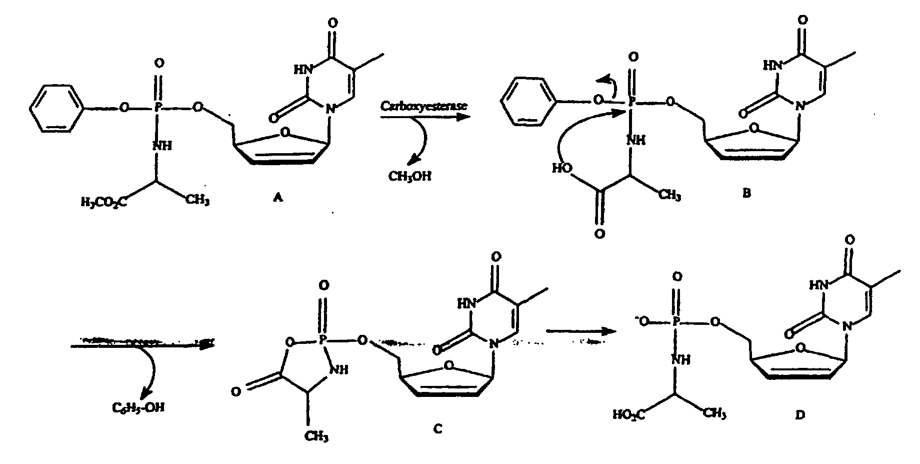

In an attempt to overcome the dependence of ddN analogues on intracellular nucleoside kinase activation, McGuigan et al. have prepared aryl methoxyalaninyl phosphate derivatives of AZT (McGuigan et al., 1993 *J. Med. Chem*. 36:1048; McGuigan et al., 1992 *Antiviral Res.* 17:311) and d4T (McGuigan et al., WO 96/29336, McGuigan et al., 1996 *J.Med.Chem*.39:1748; McGuigan et al., 1996 *Bioorg.Med.Chem.Lett*. 6:1183). Similarly, Siddiqui et aL have prepared phosphoramidate d4t-MP prodrugs for use against HIV (Siddiqui et al., *J.Med*. *Chem*. 1999, 42(20). Such compounds have been shown to undergo intracellular hydrolysis to yield monophosphate derivatives that are further phosphorylated by thymidylate kinase to give the bioactive triphosphate derivatives in a thymidine kinase (TK)-independent fashion. However, all attempts to date to further improve the potency of the aryl phosphate derivatives of d4T by various substitutions of the aryl moiety without concomitantly enhancing their cytotoxicity have failed. (McGuigan et al., 1996 *J.Med.Chem*.39:1748).

What is needed in the art is one or more useful therapeutic agents, which are potent and selective inhibitors of HIV RT. Further, what is needed in the art is one or more useful therapeutic agents, which have improved potency without concomitantly enhancing their cytotoxicity.

### Summary of the Invention

It has been discovered that the positioning of specific substituents on the aryl moiety in the aryl phosphate derivatives of nucleosides enhances the ability of the nucleoside derivatives of d4T to undergo hydrolysis due to the properties of the substituent. The substituted phenyl phosphate nucleoside derivatives of the present invention demonstrate improved potency and specific antiviral activity compared to known therapeutic agents.

Accordingly, in one aspect the present invention relates to a compound having the following formula: 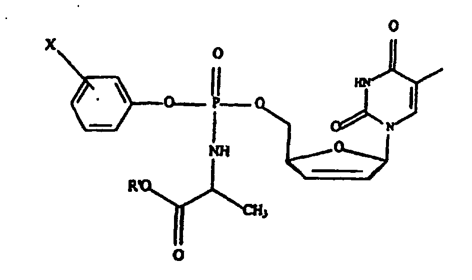 wherein R' is methyl or ethyl; X is selected from the group consisting of: 3-N(CH₃)₂; 2,6-(CH₃O)₂; 4-Br, 2-Cl; 2-Br; and 2,5-(Cl)₂; or a pharmaceutically acceptable salt thereof.

Preferably the compound of the present invention is selected from:
5'-[3-dimethylaminophenyl methoxyalaninyl phosphate]-2',3'-didehydro-3'-deoxythymidine;
5'-[2,6-dimethoxyphenyl methoxyalaninyl phosphate]-2',3'-didehydro-3'-deoxythymidine;
5'-[4-bromo-2-chlorophenyl methoxyalaninyl phosphate]-2',3'-didehydro-3'-deoxythymidine;
5'-[2-bromophenyl methoxyalaninyl phosphate]-2',3'-didehydro-3'-deoxythymidine; and
5'-[2,5-dichlorophenyl methoxyalaninyl phosphate]-2',3'-didehydro-3'-deoxythymidine and pharmaceutically acceptable salts thereof.

In another aspect, the present invention is directed to use of a compound of the invention for preparation of a medicament for viral infections. In another aspect, the invention is directed to aryl phosphate nucleoside derivatives, particularly aryl phosphate derivatives of d4T, that exhibit antiviral activity. For example, certain compounds exhibit potent activity against HIV, e.g. as inhibitors of HIV reverse transcriptase. Aryl phosphate derivatives of d4T having one or more specific substituents on the aryl group, were unexpectedly found to show markedly increased potency as anti-HIV agents without undesirable levels of cytotoxic activity. In particular, these derivatives are potent inhibitors of HIV reverse transcriptase.

The present invention is further directed to use of a compound of the invention for preparation of a medicament for inhibiting HIV reverse transcriptase in cells infected with HIV. In addition, the present invention is directed to use of a compound of the invention for preparation of a medicament for inhibiting HIV replication in a host cell.

The present invention is further directed to a pharmaceutical composition comprising an effective antimicrobial amount of a compound of the invention and one or more pharmaceutically acceptable carriers or diluents.

These and other features and advantages of the present invention will become apparent after a review of the following detailed description of the disclosed embodiments and the appended claims.

### Detailed Description of the Invention

It has been discovered unexpectedly that certain substituted aryl phosphate derivatives of nucleosides possess increased activity against HIV while maintaining low levels of cytotoxicity. As such, these derivatives are particularly useful as active agents for antiviral compositions and for methods of treating viral infections such as HIV infections.

### Compounds of the Present Invention

The compounds of the present invention, as discussed more fully in the Examples below, are aryl phosphate derivatives of nucleosides, particularly derivatives of d4T, having antiviral activities. A nucleoside derivative suitable for use in compositions and methods of the present invention is of the formula: 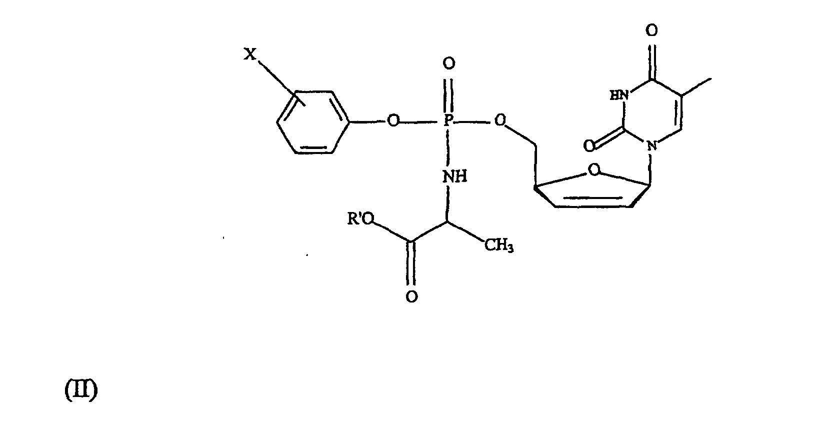 where X is selected from the group consisting of 3-N(CH₃)₂; 2,6-(CH₃O)₂; 4-Br,2-Cl; 2-Br and 2,5-(Cl)₂; and R' is methyl ethyl, or a pharmaceutically acceptable salt thereof.

In a further embodiment of the present invention, the nucleoside d4T derivatives suitable for use in compositions and methods of the present invention include:
5'-[3-dimethylaminophenyl methoxyalaninyl phosphate]-2',3'-didehydro-3'-deoxythymidine;
5'-[2,6-dimethoxyphenyl methoxyalaninyl phosphate]-2',3'-didehydro-3'-deoxythymidine;
5'-[4-bromo-2-chlorophenyl methoxyalaninyl phosphate]-2',3'-didehydro-3'-deoxythymidine;
5'-[2-bromophenyl methoxyalaninyl phosphate]-2',3'-didehydro-3'-deoxythymidine;
5'-[2,5-dichlorophenyl methoxyalaninyl phosphate]-2',3'-didehydro-3'-deoxythymidine;
or a pharmaceutically acceptable salt of any one of the compounds above.

### Synthesis of the d4T derivatives:

To generally illustrate the synthesis of compounds of the present invention, the synthesis of d4T derivatives is described below. Appropriately substituted phenyl phosphorodichloridate may be prepared by the procedures discussed in McGuigan, et al., *Antiviral Res*., 1992, 17:311.

One possible method of making the d4T derivatives of the present invention is given in Scheme 1 below:

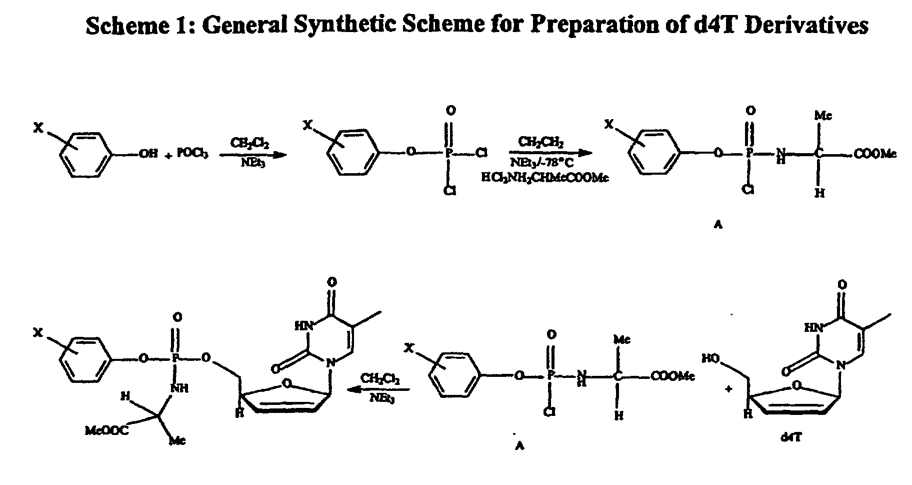

As shown in Scheme 1, a substituted phenol reacts with phosphorous oxychloride to obtain a substituted phenyl phosphorodichloridate. The substituted phenyl phosphorodichloridate further reacts with L-alanine methyl ester to form a substituted phenyl methoxyalaninyl phosphate, designated "A" in Scheme 1. It should be noted that the substituents "X" shown above in Scheme 1 represent one or more substituents on the phenyl group of the reactants.

The substituted phenyl methoxyalaninyl phosphate reacts with d4T as shown in Scheme 1 above. The substituted phenyl methoxyalaninyl phosphate (A) and d4T reacts in dichloromethane and triethylamine to form the desired products of the present invention (see Scheme 1 above).

### Administering the d4T derivatives:

The d4T derivatives may be administered to patients in the form of a suitable composition containing the d4T derivative as an active agent along with a pharmaceutically acceptable carrier, adjuvant, or diluent. The compositions may be administered either orally or parenterally. Compositions include, for example, tablets, capsules, and solutions or dispersions in vials for parenteral administration. Sustained release dosage forms may be used if desired. The compositions are administered to a patient in need of the antiviral activity in a suitable antiviral amount, for example, sufficient to inhibit the HIV reverse transcriptase and/or inhibit replication of HIV in host cells. The dose is administered according to a suitable dosage regimen.

In one embodiment of the present invention, the d4T-derivative is administered at a dosage of from about 0.1 mg/kg to about 100 mg/kg (mg of d4T per kg of body weight). Preferred methods of administering the d4T derivative include oral or intravenous delivery. A dosage may be administered for a period of seven to 30 days per course, with the number of courses varying from one to about twelve per year.

The present invention is described above and further illustrated below by way of examples, which are not to be construed in any way as imposing limitations upon the scope of the invention. On the contrary, it is to be clearly understood that resort may be had to various other embodiments, modifications, and equivalents thereof which, after reading the description herein, may suggest themselves to those skilled in the art without departing from the spirit of the present invention and/or the scope of the appended claims.

### EXAMPLE 1

### Synthesis and Characterization of d4T Derivatives

Substituted phenyl phosphorodichloridates were produced using the reaction mechanism shown in Scheme 1 above. Select "X" substituents were used to produce five substituted phenyl phosphorodichloridates. The substituted phenyl phosphorodichloridates were reacted with alanine methyl ester to form five substituted phenyl methoxyalaninyl phosphates as shown in Scheme 1. The substituted phenyl methoxyalaninyl phosphates were then reacted with d4T as shown in Scheme 1 above to form five d4T derivatives of the present invention.

The following compounds were produced using the general procedure outlined above:
**Compound 1**: 5'-[3-dimethylaminophenyl methoxyalaninyl phosphate]-2',3'-didehydro-3'-deoxythymidine;
**Compound 2**: 5'-[2,6-dimethoxyphenyl methoxyalaninyl phosphate]-2',3'-didehydro-3'-deoxythymidine;
**Compound 3**: 5'-[4-bromo-2-chlorophenyl methoxyalaninyl phosphate]-2',3'-didehydro-3'-deoxythymidine;
**Compound 4**: 5'-[2-bromophenyl methoxyalaninyl phosphate]-2',3'-didehydro-3'-deoxythymidine; and
**Compound 5**: 5'-[2,5-dichlorophenyl methoxyalaninyl phosphate]-2',3'-didehydro-3'-deoxythymidine.

Melting points were determined using a Fisher-Johns melting apparatus and are uncorrected. ¹H NMR spectra were recorded using a Varian Mercury 300 spectrometer in DMSO-d₆ or CDCl₃. Chemical shifts are reported in parts per million (ppm) with tetramethylsilane (TMS) as an internal standard at zero ppm. Infrared spectra were recorded on a Nicolet PROTEGE 460-IR spectrometer. Mass spectroscopy data were recorded on a FINNIGAN MAT 95, VG 7070E-HF G.C. system with an HP 5973 Mass Selection Detector. UV spectra were recorded on BECKMAN DU 7400 and using MeOH as the solvent. TLC was performed on a precoated silica gel plate (Silica Gel KGF; Whitman Inc). Silica gel (200-400 mesh, Whitman Inc.) was used for all column chromatographic separations. HPLC was performed using a C18 4 x 250 mm LiChrospher column eluted with 70:30 water/acetonitrile at the flow rate of 1 mL/min. The purity of the following compounds exceeded 96% by HPLC. All chemicals were reagent grade and were purchased from Aldrich Chemical Company (Milwaukee, Wis) or Sigma Chemical Company (St. Louis, MO).

### Physical constant section:

**Compound 1:** Yield: 0.83 g (18%); mp 61-62 °C; ¹H NMR (CDCl₃) δ 9.93 (s, 1 H), 7.27 (br d, *J* = 20.1 Hz, 1 H), 7.04 (m, 1 H), 6.97 (m, 1 H), 6.44 (m, 3 H), 6.24 (dd, *J*= 6.0, 22.2 Hz, 1H), 5.81 (m, 1 H), 4.94 (m, 1 H), 4.24 (s, 2 H), 4.08 (m, 1 H), 3.92 (m, 1 H), 3.64* (d, *J* = 1.2 Hz, 3 H), 2.86 (s, 6 H), 1.77* (d, *J* = 6.0 Hz, 3 H), 1.28* (m, 3 H); ¹³C NMR (CDCl₃) δ 173.7*, 163.9*, 151.3*, 150.8*, 135.5*, 132.9*, 129.5*, 126.9*, 111.0*, 108.8*, 107.2*, 103.7*, 89.3*, 84.4*, 66.7*, 66.1*, 52.3*, 49.9*, 40.2, 20.7*, 12.2; ³¹P NMR (CDCl₃) δ 3.32, 2.70; IR (KBr) ν 3448, 3050, 2952, 1691, 1506, 1450, 1247, 1143, 999 cm⁻¹; UV λₘₐₓ 203, 206, 21, 258 nm; FAB MS *m*/*z* 531.1619 (C₂₂H₂₉N₄O₈P + Na⁺); HPLC t_{R} 3.36 min.
**Compound 2:** Yield: 0.60 g (13%); mp: 51-53 °C; ¹H NMR (CDCl₃) δ 9.78 (s, 1 H), 7.38 (br d, *J* = 21.3 Hz, 1 H), 6.95 (m, 3 H), 6.48 (m, 3 H), 6.29 (dd, *J* = 6.0, 22.0 Hz, 1 H), 5.81 (d, *J* = 5.4 Hz, 1 H), 4.36 (m, 3 H), 4.02 (m, 2 H), 3.74 (d, *J*= 9.3 Hz, 6 H), 3.63* (d, *J* = 4.2 Hz, 3 H), 1.74* (d, *J* = 8.1 Hz, 3 H), 1.29* (m, 3 H); ¹³C NMR (CDCl₃) δ 173.7*, 163.9*, 151.7*, 150.8*, 135.7*, 133.1*, 128.4, 126.8*, 125.0*, 110.9*, 104.8*, 89.2, 84.6*, 66.8*, 55.8*, 52.2*,49.7*, 49.4*, 21.0*, 11.8*; ³¹P NMR (CDCl₃) δ 4.97, 4.28; IR (KBr) ν 3432, 3072, 2950, 1691, 1483, 1261, 1112, 931 cm⁻¹; UV λₘₐₓ 210,267 nm; FAB MS *m*/*z* 526.1570 (C₂₂H₂₈N₃O₁₀P + H⁺); HPLC t_{R} 6.55 min.
**Compound 3:** Yield: 0.89 g (17%); mp: 51-52 °C; ¹H NMR (CDCl₃) δ 9.52 (s, 1 H), 7.52 (s, 1 H), 7.32 (m, 2 H), 7.22 (dd, *J* = 1.2, 17.4 Hz, 1 H), 6.99 (m, 1 H), 6.29 (dd, *J* = 6.0, 14.7 Hz, 1 H), 5.90 (d, *J* = 6.0 Hz, 1 H), 5.00 (m, 1 H), 4.33 (m, 2 H), 4.19 (m, 1 H), 4.01 (m, 1 H), 3.67 (s, 1 H), 1.79* (d, *J* = 14.1 Hz, 3 H), 1.31 * (dd, *J* = 7.2, 10.5 Hz, 3 H); ¹³C NMR (CDCl₃) δ 173.5*, 163.8*, 150.8, 145.5*, 135.3*, 132.8*, 130.9, 127.3*, 126.2*, 122.7*, 117.8*, 113.3*, 89.6*, 84.3*, 67.5*, 67.1*, 52.6, 50.1, 20.8*, 12.3*; ³¹P NMR (CDCl₃) δ 3.11, 2.54; IR (KBr) ν 3415, 3222, 3072, 2952, 1691, 1475, 1245, 1085, 1035, 929 cm⁻¹; UV λₘₐₓ 215, 267 nm; FAB MS *m*/*z* 578.0105 (C₂₀H₂₂BrClN₃O₈P + H⁺); HPLC t_{R} 18.63, 20.63 min.
**Compound 4:** Yield: 0.36 g (19%); mp: 45-46 °C; ¹H NMR (CDCl₃) δ 9.55 (s, 1 H), 7.47 (m, 2 H), 7.24 (m, 2 H), 6.99 (m, 2 H), 6.29 (dd, *J* = 6.0, 16.8 Hz, 1 H), 5.88 (m, 1 H), 5.00 (m, 1 H), 4.35 (m, 2 H), 4.02 (m, 2 H), 3.66 (s, 3 H), 1.80* (d, *J* = 13.2 Hz, 3 H), 1.30* (dd, *J* = 6.6, 15.3 Hz, 3 H); ¹³C NMR (CDCl₃) δ 173.6*, 163.8*, 150.8, 147.3*, 135.4*, 133.0*, 128.5*, 127.2*, 126.1, 121.3*, 114.4*, 111.3*, 89.6*, 84.3*, 67.2*, 52.5, 50.1*, 29.6, 20.8*, 12.4; ³¹P NMR (CDCl₃) δ 2.98, 2.37; IR (KBr) ν 3432, 3072, 2954, 1685, 1475, 1245, 1089, 933 cm⁻¹; UV λₘₐₓ 207, 267 nm; FAB MS *m*/*z* 544.0469 (C₂₀H₂₃BrN₃O₈P + H⁺); HPLC t_{R} 8.37, 9.23 min.
**Compound 5:** Yield: 0.68 g (30%); mp: 42-44 °C; ¹H NMR (CDCl₃) δ 9.43 (s, 1 H), 7.45 (m, 1 H), 7.25 (m, 2 H), 7.04 (dd, *J* = 2.4, 8.7 Hz, 1 H), 6.99 (m, 1 H), 6.32 (m, 1 H), 5.88 (m, 1 H), 4.99 (m, 1 H), 4.32 (m, 3 H), 4.00 (m, 1 H), 3.67 (s, 3 H), 1.77* (dd, *J* = 1.2, 19.8 Hz, 3 H), 1.33* (m, 3 H); ¹³C NMR (CDCl₃) δ 173.5*, 163.8, 150.8, 146.4*, 135.3, 132.7*, 130.7*, 127.4, 125.8, 123.7*, 121.7*, 111.2*, 89.6*, 84.3*, 67.1*, 52.6, 50.1, 29.6, 20.7*, 12.3*; ³¹P NMR (CDCl₃) δ 3.24, 2.60; IR (KBr) ν 3423, 3205, 3072, 2954, 1691, 1475, 1245, 1093, 946 cm⁻¹; UV λₘₐₓ 211, 216, 220, 268 nm; FAB MS *m*/*z* 534.0581 (C₂₀H₂₂Cl₂N₃O₈P + H⁺); HPLC t_{R} 13.18 min.
* multiple peaks are observed due to isomers

### EXAMPLE 2

### Intracellular metabolism of compounds 1-5 in PBMC cells

The compounds, as well as AZT, were tested for their ability to inhibit HIV replication in peripheral blood mononuclear cells using previously described procedures (Zarling et al., 1990 *Nature* 347:92; Erice et al., 1993 *Antimicrob. Agents Chemother.* 37:835; Uckun et. al., 1998 *Antimicrob. Agents Chemother.* 42:383). Anti-HIV activities were evaluated in AZT-sensitive HIV-1(strain: HTLVIIIB) infected peripheral blood mononuclear cells (PBMC) by determining the concentration of compound needed to inhibit viral replication by 50%, based on reverse transcriptase activity assays (IC₅₀). Percent viral inhibition was calculated by comparing the RT activity values from the test substance-treated infected cells with RT values from untreated infected cells (i.e., virus controls).

In parallel, the cytotoxicity of the compounds was examined using a microculture tetrazolium assay (MTA) of cell proliferation, as described (in the Zarling Enrice, and Uckun articles *Supra*). More specifically, the 50% cytotoxic concentrations of the compounds (CC₅₀) were measured by MTA, using 2,3-bis(2-methoxy-4-nitro-5-sulfophenyl)-5-[(phenylamino)-carbonyl]-2H-tetrazolium hydroxide (XTT) (Zarling et al., 1990; Erice et al., 1993, Uckun et al., 1998, *Supra*).

The results are shown in **Tables 1** and **2,** wherein each Table of results represents a separate experiment using different PBMC.

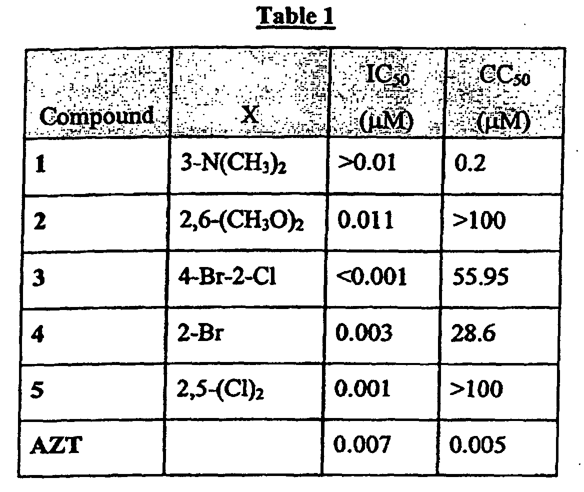

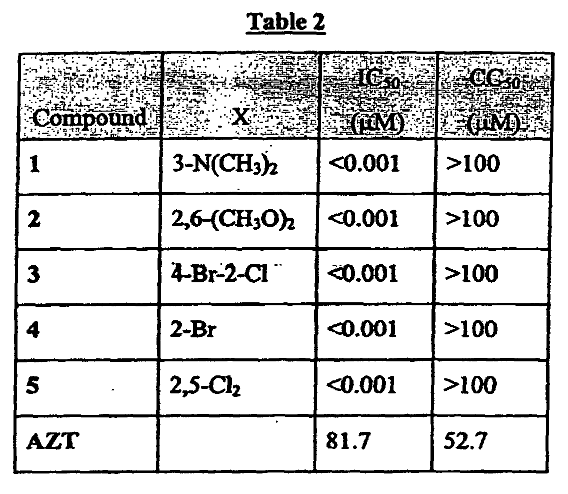

## Claims

1. A compound having the following formula: 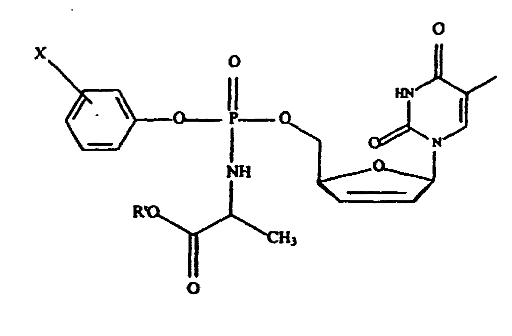 wherein R' is methyl or ethyl; X is selected from the group consisting of: 3-N(CH₃)₂; 2,6-(CH₃O)₂; 4-Br,2-Cl; 2-Br; and 2,5-(Cl)₂; or a pharmaceutically acceptable salt thereof.

2. The compound of Claim 1, wherein X is 3-N(CH₃)₂.

3. The compound of Claim 1, wherein X is 2,6-(CH₃O)₂.

4. The compound of Claim 1, wherein X is 4-Br,2-Cl.

5. The compound of Claim 1, wherein X is 2-Br.

6. The compound of Claim 1, wherein X is 2,5-(Cl)₂.

7. The compound of Claim 1, and being 5'-[3-dimethylaminophenyl methoxyalaninyl phosphate]-2',3'-didehydro-3'-deoxythymidine or a pharmaceutically acceptable salt thereof.

8. The compound of Claim 1, and being 5'-[2,6-dimethoxyphenyl methoxyalaninyl phosphate]-2',3'-didehydro-3'-deoxythymidine or a pharmaceutically acceptable salt thereof.

9. The compound of Claim 1, and being 5'-[4-bromo-2-chlorophenyl methoxyalaninyl phosphate]-2',3'-didehydro-3'-deoxythymidine or a pharmaceutically acceptable salt thereof.

10. The compound of Claim 1, and being 5'-[2-bromophenyl methoxyalaninyl phosphate]-2',3'-didehydro-3'-deoxythymidine or a pharmaceutically acceptable salt thereof.

11. The compound of Claim 1, and being 5'-[2,5-dichlorophenyl methoxyalaninyl phosphate]-2',3'-didehydro-3'-deoxythymidine or a pharmaceutically acceptable salt thereof.

12. Use of a compound according to any of claims 1 to 11 for preparation of a medicament for treating viral infections.

13. Use of a compound according to any of claims 1 to 11 for preparation of a medicament for inhibiting HIV reverse transcriptase.

14. Use of a compound according to any of claims 1 to 11 for the preparation of a medicament for inhibiting HIV replication in host cells.

15. A pharmaceutical composition comprising an effective antimicrobial amount of a compound according to any of claims 1 to 11 and one or more pharmaceutically acceptable carriers or diluents.

## Patentansprüche

1. Verbindung mit der folgenden Formel: 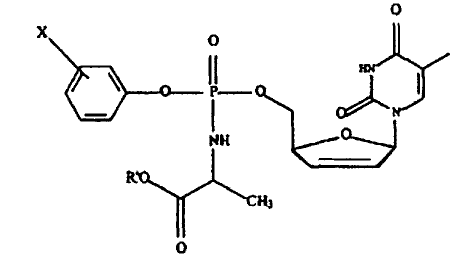 worin R' Methyl oder Ethyl ist; X aus der Gruppe ausgewählt ist, die 3-N-(CH₃)₂, 2,6-(CH₃O)₂, 4-Br, 2-Cl, 2-Br und 2,5-(Cl)₂ bedeutet, oder ein pharmazeutisch verträgliches Salz hiervon.

2. Verbindung nach Anspruch 1, worin X 3-N-(CH₃)₂ ist.

3. Verbindung nach Anspruch 1, worin X 2,6-(CH₃O)₂ ist.

4. Verbindung nach Anspruch 1, worin X 4-Br, 2-Cl ist.

5. Verbindung nach Anspruch 1, worin X 2-Br ist.

6. Verbindung nach Anspruch 1, worin X 2,5-(Cl)₂ ist.

7. Verbindung nach Anspruch 1 der Formel 5'-[3-Dimethylaminophenyl-methoxyalaninylphosphat]-2',3'-didehydro-3'-deoxythymidin oder ein pharmazeutisch verträgliches Salz hiervon.

8. Verbindung nach Anspruch 1 der Formel 5'-[2,6-Dimethoxyphenylmethoxyalaninylphosphat]-2',3'-didehydro-3'-deoxythymidin oder ein pharmazeutisch verträgliches Salz hiervon.

9. Verbindung nach Anspruch 1 der Formel 5'-[4-Brom-2-chlorphenylmethoxyalaninylphosphat]-2',3'-didehydro-3'-deoxythymidin oder ein pharmazeutisch verträgliches Salz hiervon.

10. Verbindung nach Anspruch 1 der Formel 5'-[2-Bromophenylmethoxyalaninylphosphat]-2',3'-didehydro-3'-deoxythymidin oder ein pharmazeutisch verträgliches Salz hiervon.

11. Verbindung nach Anspruch 1 der Formel 5'-[2,5-Dichlorphenylmethoxyalaninylphosphat]-2',3'-didehydro-3'-deoxythymidin oder ein pharmazeutisch verträgliches Salz hiervon.

12. Verwendung einer Verbindung nach einem der Ansprüche 1 bis 11 zur Herstellung eines Arzneimittels für die Behandlung viraler Infektionen.

13. Verwendung einer Verbindung nach einem der Ansprüche 1 bis 11 zur Herstellung eines Arzneimittels zur Hemmung von HIV-Umkehrtranskriptase.

14. Verwendung einer Verbindung nach einem der Ansprüche 1 bis 11 zur Herstellung eines Arzneimittels zur Hemmung von HIV-Replikation in Wirtszellen.

15. Pharmazeutische Zusammensetzung mit einer wirksamen antimikrobiellen Menge einer Verbindung nach einem der Ansprüche 1 bis 11 und einem oder mehreren pharmazeutisch verträglichen Trägern oder Verdünnungsmitteln.

## Revendications

1. Composé de formule suivante : 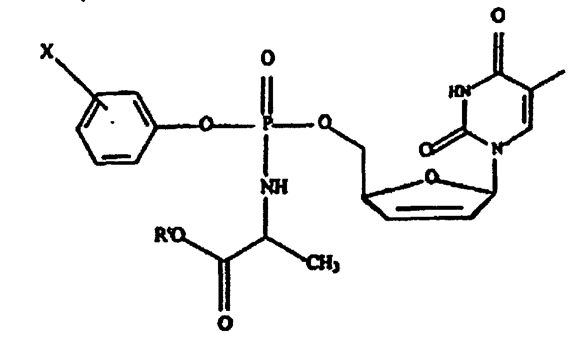 dans laquelle R' représente un groupe méthyle ou éthyle ; X est choisi dans le groupe consistant en : 3-N(CH₃)₂ ; 2,6-(CH₃O)₂ ; 4-Br,2-Cl ; 2-Br et 2,5-(Cl)₂ ; ou un de ses sels pharmaceutiquement acceptables.

2. Composé suivant la revendication 1, dans lequel X représente 3-N(CH₃)₂.

3. Composé suivant la revendication 1, dans lequel X représente 2,6-(CH₃O)₂.

4. Composé suivant la revendication 1, dans lequel X représente 4-Br,2-Cl.

5. Composé suivant la revendication 1, dans lequel X représente 2-Br.

6. Composé suivant la revendication 1, dans lequel X représente 2,5-(Cl)₂.

7. Composé suivant la revendication 1, qui est la 5'-[3-diméthylaminophénylméthoxyalaninylphosphate]-2',3'-didéshydro-3'-désoxythymidine ou un de ses sels pharmaceutiquement acceptables.

8. Composé suivant la revendication 1, qui est la 5'-[2,6-diméthoxyphénylméthoxyalaninylphosphate]-2',3'- didéshydro-3'-désoxythymidine ou un de ses sels pharmaceutiquement acceptables.

9. Composé suivant la revendication 1, qui est la 5'-[4-bromo-2-chlorophénylméthoxyalaninylphosphate]-2',3'-didéshydro-3'-désoxythymidine ou un de ses sels pharmaceutiquement acceptables.

10. Composé suivant la revendication 1, qui est la 5'-[2-bromophénylméthoxyalaninylphosphate]-2',3'-didéshydro-3'-désoxythymidine ou un de ses sels pharmaceutiquement acceptables.

11. Composé suivant la revendication 1, qui est la 5'-[2,5-dichlorophénylméthoxyalaninylphosphate]-2',3'-didéshydro-3'-désoxythymidine ou un de ses sels pharmaceutiquement acceptables.

12. Utilisation d'un composé suivant l'une quelconque des revendications 1 à 11 pour la préparation d'un médicament destiné au traitement d'infections virales.

13. Utilisation d'un composé suivant l'une quelconque des revendications 1 à 11 pour la préparation d'un médicament destiné à inhiber la transcriptase inverse du VIH.

14. Utilisation d'un composé suivant l'une quelconque des revendications 1 à 11 pour la préparation d'un médicament destiné à inhiber la réplication du VIH dans des cellules hôtes.

15. Composition pharmaceutique comprenant une quantité antimicrobienne efficace d'un composé suivant l'une quelconque des revendications 1 à 11 et un ou plusieurs supports ou diluants pharmaceutiquement acceptables.
